# EUROPEAN PATENT SPECIFICATION

(11) **EP 0 735 139 B1**
(45) Date of publication and mention of the grant of the patent: **01.10.2003**
(21) Application number: 96102098.9
(22) Date of filing: 20.06.1990
(51) Int. Cl.: C12N 15/16

(54) **Heteropolymeric protein production**
Herstellung von heteropolymeren Proteinen
Production de protéines hétéropolymères

(30) Priority: 20.06.1989 US 368628
(43) Date of publication of application: 02.10.1996
(62) Divisional of application: 90306743.7
(73) Proprietor: Applied Research Systems ARS Holding N.V., Curacao (AN)
(72) Inventor: Kelton, Christie A., Hopkinton, Massachusetts 01748 (US); Nugent, Noreen P., Framingham, Massachusetts 01701 (US); Chappel, Scott C., 02186 Milton, Massachusetts (US)
(74) Representative: White, Martin Paul

(56) References cited:
- WO-A-90/08528
- THE JOURNAL OF CELL BIOLOGY, vol. 106, no. 4, April 1988, pages 1049-1059, XP002009362 M.M.MATZUK AND I.BOIME: "The role of the asparagin-linked oligosaccharides of the alpha subunit in the secretion and assembly of human chorionic gonadotropin"
- PROCEEDINGS OF THE NATIONAL ACADEMY OF SCIENCES OF USA, vol. 82, November 1985, WASHINGTON US, pages 7280-7283, XP002009363 D.KAETZEL ET AL.: "Expression of biologically active bovine luteinizing hormone in Chinese Hamster Ovary cells"

## Description

This invention relates to methods for the production of heteropolymeric dimeric protein selected from luteinising hormone, follicle stimulating hormone or chorionic gonadotropin, from transformed mammalian cells.

Transfection of the α and β subunit cDNA clones into cultured mammalian cells has characteristically resulted in low gonadotropin expression levels. This has seriously impeded the production of these hormones on a commercial scale.

It is one aspect of the present invention to provide commercially practical methods for the production of such hormones.

While some genes, such as β globin (1, 2) and immunoglobulin genes (3-5), require introns for optimal mRNA production, other genes, such as thymidine kinase (6), do not. Intron-dependent increases in gene expression can result from either non-non-transcriptional (e.g. globin genes) or transcriptional (e.g. immunoglobulin genes) mechanisms.

Isolation of the genes which encode the.human and bovine common α, FSH β, LH β, and human TSHβ subunits has been reported (8-14). Ramabhadran et al. (15) first described transfection with and subsequent expression of the human alpha subunit cDNA in mouse cells. Several groups have since reported successful expression of dimericglycoprotein hormones by transfection of cultured mammalian cells. Some of these groups (16,17) employed cDNA clones while others (14, 18, 19) have used intro-containing cDNA or genomic sequences.

WO 85/01958 describes the use of cDNA clones (without introns) to produce gonadotropins in cultured mammalian cells. While those expression systems yield biologically active molecules, the yield of the transformed mammalian cells is generally lower than described.

It is another aspect of the present invention to provide improved expression systems, useful with, for example, gonadotropins, which result in higher yield.

Matzuk and Boime (18a) mention that an intron inserted into the coding region of the human α subunit cDNA improved expression results compared with the use of cDNA clones but provided no data to support that contention or specific description of their methods. In a recent publication, Kaetzel and Nilson (7) reported relatively high levels of bovine LH expression in CHO cells. Their system employed genomic sequences for expression of both the α and LHβ subunits. However, the effect of genomic sequences versus cDNA sequences upon LH expression was not addressed in their paper.

It is yet another aspect of the present invention to obviate the confusion represented by the present state of the art and to provide the critical teaching necessary to derive improved vectors encoding dimeric glycoproteins and production methods utilizing such vectors.

Introns have been linked to increased mRNA accumulation in tissue culture cells for rabbit β-globin (1,2,20), E. coli gpt (20), and mouse DHFR (20, 21). Examples of genes containing introns with enhancer elements which increase transcription are the immunoglobulin genes (3-5), the rat cytochrome c gene (22), and the human pro-α1(I) collagen gene (23). Introns have also been shown to result in increased transcriptional efficiency in transgenic mice for the following genes: rat growth hormone, mouse metallothionein-I, and human β-globin (24). However, introns have no effect on the expression of these last three genes when they are transfected into cultured mammalian cells.

It has been shown that expression levels can be influenced by different 3' untranslated and polyadenylation regions (24, 25). For example, higher expression levels of a galK marker gene result if the bovine growth hormone polyadenylation region is used for transcription termination rather than the SV40 early or human collagen polyadenylation regions (24).

In accordance with the various principles and aspects of the present invention there are provided novel methods employing the use of α subunit genomic sequences, or α subunit cDNA constructions with an added intron, which significantly and suprisingly enhance dimeric glycoprotein hormone production in mammalian cells. This discovery will facilitate the development of processes for high-yield production of dimeric glycoprotein hormones which share the common α subunit. These include chorionic gonadotropin (CG), follicle stimulating hormone (FSH) and luteinizing hormone (LH).

Preferred embodiments and examples of the present invention will now be described with reference to the accompanying drawings in which:
Figure 1A+B: shows the strategy used to engineer the human α gene for expression in tissue culture cells. Important restriction endonuclease sites are indicated.
   Filled-in boxes represent α gene exons; heavy solid lines, α gene introns; thin solid lines, pBR322 or pUC18 vector; and zig-zag lines, pUC18 polylinker regions.
Figure 2: shows the basic expression vector, CLH3AXSV2[DHFR, ODC, or TPA], used for stable or transient transfections. The position of the XhoI site used for insertion of α or β subunit sequences is shown.
   Double solid lines represent sequences needed for mammalian cell expression. The relative positions of the promoter, polyadenylation, and marker gene regions are indicated. The single solid line represents the pBR322-derived pML-1 region necessary for propagation and selection (by ampicillin resistance) in E. coli.

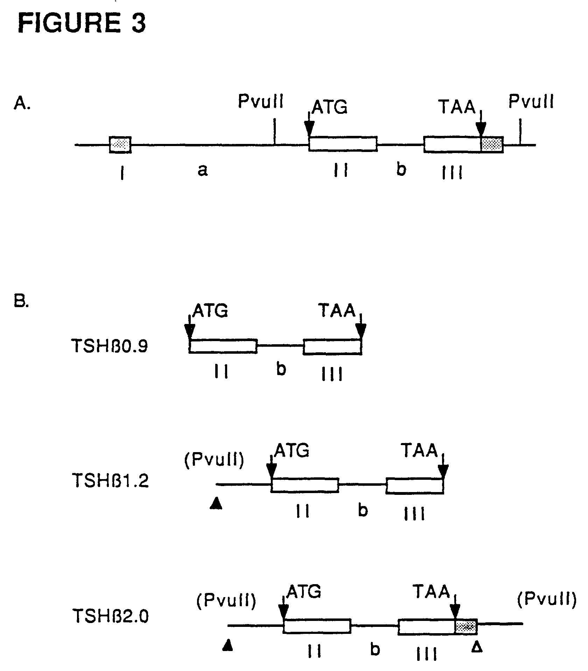

Figure 3: part A shows the full-length TSHβ gene. The positions of the three exons (I, II, and III), two introns (a, b), start ATG, and stop codon (TAA) are indicated. The PvuII sites used to isolate the fragment from which the partial constructions were derived are also indicated. Hatched boxes denote noncoding exon sequences. Part B shows the partial genomic constructions used to compare TSHβ mRNA accumulation in transiently transfected COS-7 cells. TSHβ 0.9 consists of the two coding exons separated by the endogenous IVS with all sequences upstream of the start ATG and downstream of the TAA removed (sequence shown in Table 9). TSHβ 1.2 and TSHβ 2.0 contain, in addition, about 300 base pairs of intron a and were constructed by adding a synthetic splice donor (▲) to allow splicing of the truncated intron. TSHβ 2.0 retained the endogenous polyadenylation signal (Δ) and about 0.8 kb of additional 3' flanking sequence.

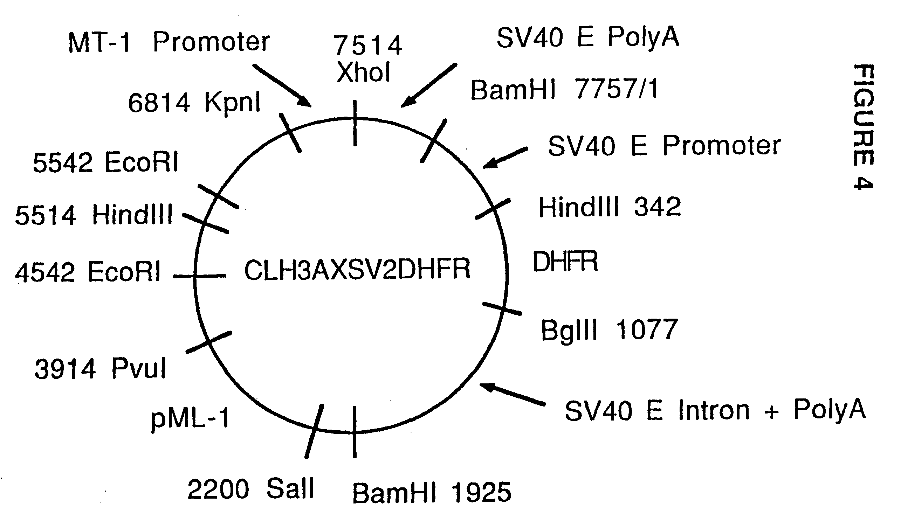

Figure 4: describes CLH3AXSV2DHFR. This vector was constructed from the following components: (i) the dehydrofolate reductase (DHFR) transcriptional unit (nucleotide numbers 1 to 1925 of Figure 4) which consists of the SV40 early region promoter (33, 34), the mouse DHFR gene (REFS) and the SV40 small T intron and early region polyadenylation signal sequences (33, 34); (ii) the bacterial plasmid vector sequences of pML (nucleotide numbers 2201 to 4542 of Figure 4) derived from the pBR322 vector (29) from which a 1370 base pair sequence has been deleted (32); and (iii) the metallothionein promoter (nucleotide numbers 4542 to 7514 of Figure 4) derived from the mouse metallothionein-1 gene (30, 31) from which the introns and polyadenylation signal sequences have been removed; and (iv) the SV40 early region polyadenylation signal sequences (nucleotide numbers 7514 to 7751 of Figure 4) (33, 34). The tPA analogues were inserted into the vector as a SalI fragment at the unique XhoI site. The orientation of the insert relative to the promoter and polyadenylation sequences was determined by restriction enzyme analysis.

### Example 1: Plasmid constructions for the expression of the common α and FSHβ subunits.

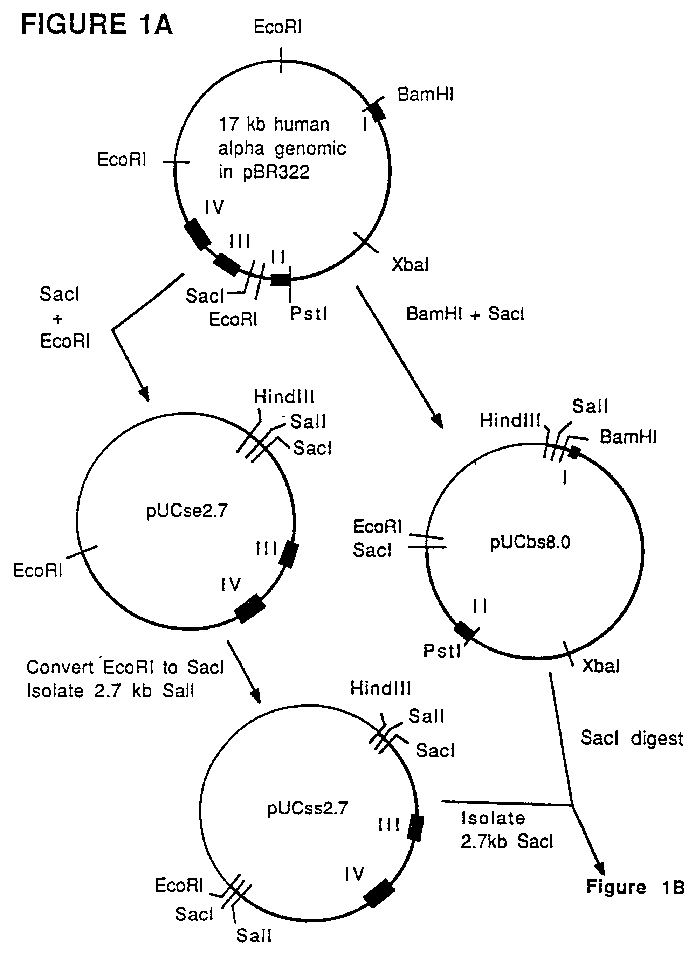

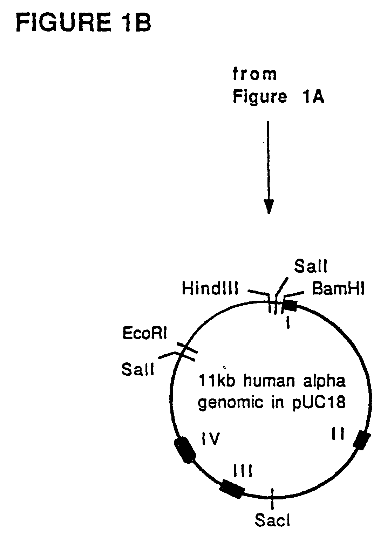

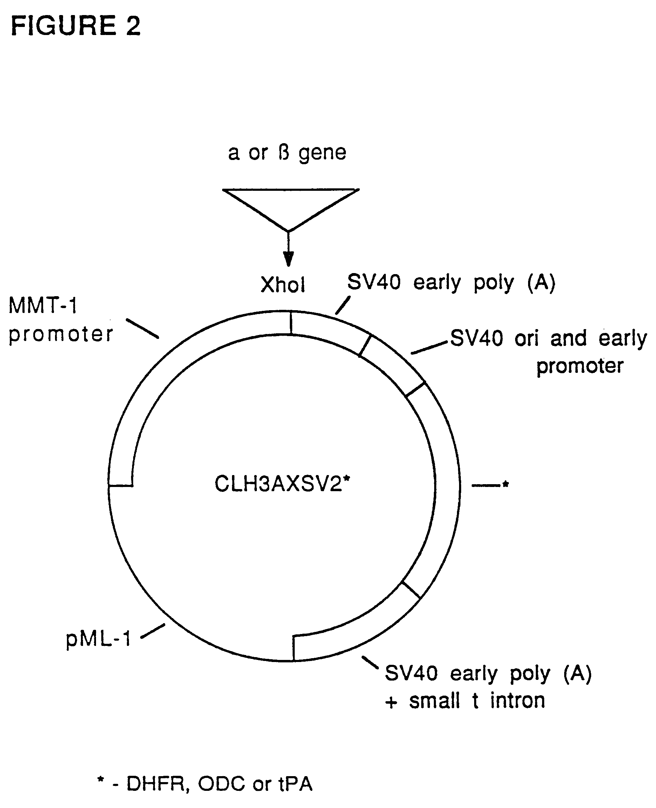

A. The complete human α subunit genomic clone can be conveniently obtained from a number of sources as a 17 kilobase pair (kb) EcoRI insert in pBR322. Because the α promoter has been shown to be tissue specific and would be unlikely to function efficiently in the tissue culture cells commonly used for heterologous gene expression, steps were taken to remove all 5' flanking sequences. The presence of internal EcoRI sites necessitated several subcloning steps prior to assembly of the trimmed genomic sequence. The engineering strategy advantageously used is diagrammed in Figure 1. Two pUC18 subclones were constructed, the first with the 8.0 kb BamHI-SacI 5' piece (pUCbs8.0), and the second with the 3' 2.7 kb SacI-EcoRI piece (pUCse2.7). To generate a terminus compatible with the XhoI cloning sites in our expression vectors, pUCse2.7 was digested with EcoRI, the ends were blunted by treatment with the Klenow fragment of E. coli DNA polymerase I, and then SalI linkers were attached in a ligation reaction. Subsequent SalI restriction endonuclease digestion of the reaction mixture yielded a 2.7 kb human α SalI piece in addition to the 2.6 kb vector fragment. The 2.7 kb human α SalI piece was gel purified and re-inserted into into the SalI site of pUC18 (pUCss2.7). A clone was chosen which contained the SalI insert in the orientation which permitted isolation of the 2.7 kb α fragment as a SacI piece. This was gel purified and then inserted into the SacI site of pUCbs8.0 to assemble the complete coding sequence of the human α gene as an 11 kb insert in pUC18. The insert could be excised as a SalI fragment by virtue of a pre-existing SalI site in the pUC18 polylinker at the 5' end of the gene and the converted SalI site (from EcoRI) at the 3' end of the gene. The completed genomic α expression construction, henceforth referred to as the "full-length genomic α" sequence, included part of exon I (less the first 35 nucleotides which comprise the 5' untranslated region of the mRNA), all of exons II, III, and IV as well as the intervening sequences, and approximately two kilobase pairs (kb) of 3' flanking sequence. This was inserted into the XhoI site of the CLH3AXSV2DHFR expression vector (Figure 2) so that transcription was directed by the mouse metallothionein-I (MT-I) promoter. The expression vector also contained the mouse dihydrofolate reductase (DHFR) gene for a selectable and amplifiable marker.
B. The human α subunit cDNA was engineered for expression by digesting the full-length clone with NcoI, which spans the start ATG, and HindIII, which cleaves in the 3' untranslated region 215 base pairs (bp) downstream of the TAA stop codon. A 5' SalI site and Kozak consensus sequence (27) was provided by synthetic oligonucleotides, and a 3' SalI site by attaching linkers as described above. The DNA sequence of the engineered α subunit cDNA clone, which is approximately 600 bp in length, is shown in Table 7. This was inserted into the XhoI site of the CLH3AXSV2DHFR expression vector (Figure 2). The endogenous 5' untranslated region and 3' polyadenylation signal were removed from the cDNA clone in the process of engineering and therefore were supplied by vector sequences: the MT-I promoter and the simian virus 40 (SV40) early polyadenylation signal, respectively.
C. The human FSHβ partial genomic clone used in this study was a 2.0 kb DdeI-Sau3A segment which contained the protein coding region of exon II in addition to 40 bp of sequence upstream of the start ATG, the protein coding region of exon III, and the 1.6 kb intron which separates the two exons (Table 7). The 5' DdeI and the 3' Sau3A sites had formerly been converted to EcoRI and BamHI sites, respectively, and therefore were not compatible with the current expression vectors. The partial FSHβ genomic clone was therefore supplied with SalI termini by blunting as described above and attaching commercially prepared SalI linkers. The SalI piece was then inserted into the XhoI site of CLH3AXSV2ODC (Figure 2), an expression vector structurally similar to CLH3AXSV2DHFR except that the DHFR coding region was replaced with that for murine ornithine decarboxylase (ODC).

### Example 2: Comparison of the full-length genomic α and cDNA α CLH3AXSV2DHFR transcription units in stable transfections.

The genomic α and cDNA α CLH3AXSV2DHFR constructions were compared by cotransfection of each α expression plasmid with the human FSHβ CLH3AXSV2ODC expression plasmid and measurement of FSH dimer production.
A. Twenty-four hours before the transfections were to be done, 100 millimeter dishes were seeded with 7 X 10⁵ DUKX CHO cells (DHFR-minus). Calcium phosphate precipitations were obtained by adding 31 microliters (µl) of 2 molar (M) CaCl₂ to the plasmid DNA suspended in 500 µl of transfection buffer [14 millimolar (mM) NaCl, 5 mM KCl, 0.7 mM Na₂HPO₄, 5.5 mM dextrose, and 20 mM HEPES (pH 7.5)]. Plasmid DNA amounts used vere α (cDNA or genomic): 10 µg, and FSHβ: 30 µg. The precipitates were allowed to form for 45 minutes at room temperature. The culture medium was removed from the cells and replaced with the DNA precipitate. After allowing the cells to sit at room temperature for 20 minutes, 5 ml of culture medium was added to each dish and incubation was continued at 37°C for 4 hours. The cells were then shocked for 3.5 minutes in 15% glycerol at 37°C. After incubation for 48 hours at 37°C in culture medium the cells were split 1:10 and selection medium containing 0.02 methotrexate (MTX) was added. The selection medium used was α-minus modified Eagle's medium supplemented with 10% dialyzed fetal bovine serum and 1% L-glutamine. Ten to fourteen days later, foci were visible and were transferred to 24-well plates. Culture media from these were assayed for FSH dimer expression by using a specific monoclonal-based radioimmunoassay (Serono Diagnostics, Randolph, MA). Positive clones were transferred to T-25 flasks in selection medium which contained an increased MTX concentration of 0.1 µM. When the cultures reached confluence the media were again assayed for FSH dimer and the cells were counted to calculate the picogram per cell per 24 hour expression levels.
B. Human FSH dimer secretion levels measured in seven randomly selected clones from each of the human α genomic/FSHβ and human α cDNA/FSHβ cotransfections are presented in Table 1.

**TABLE 1**

| α GENOMIC-DHFR/FSHβ-ODC | | α cDNA-DHFR/FSHβ-ODC | |
|---|---|---|---|
| GFSH Cell Line | pg/cell/24h in 0.1 µM MIX | CFSH Cell Line | pg/cell/24h in 0.1 µM MTX |
| 1 | 0.43 | 18 | 0.003 |
| 3 | 1.95 | 37 | 0.03 |
| 4 | 0.50 | 51 | 0.08 |
| 5 | 0.70 | 57 | 0.02 |
| 7 | 1.44 | 60 | 0.056 |
| 8 | 1.18 | 66 | 0.051 |
| 9 | 2.56 | 70 | 0.013 |
| Avg. | 1.25 | Avg. | 0.04 |

The results show that FSH dimer expression is greatly enhanced in cells transfected with the full-length genomic α subunit sequence. The averaged expression levels indicate that the surprisingly large magnitude of the enhancement seen in this particular experiment was approximately thirty-fold.
C. To further demonstrate the superiority of the full-genomic α sequence, stable cell lines were transfected with the CLH3AXSV2DHFR expression vector that contained either the human α cDNA or the human α genomic clone. Expression rates of the free α subunit were compared. In all cases, the expression of human α subunit, as determined by a sensitive and specific radioimmunoassay, was never greater than 0.05 pg/cell/24h for the cDNA-containing cell lines. As detailed in Table 2, cells that were transfected with the genomic α clone expressed 5-20 times greater levels of the protein:

**TABLE 2**

| Alpha Cell Line | Expression Level pg/cell/24h |
|---|---|
| 2 | 0.32 |
| 10 | 0.36 |
| 12 | 0.57 |
| 17 | 0.37 |
| 18 | 0.39 |
| 38 | 1.28 |
| 47 | 0.63 |
| 51 | 0.26 |

### Example 3: Thyroid Stimulating Hormone Constructions

To demonstrate the effectiveness and broad application of this invention, stably transfected CHO cell lines were prepared by cotransfection of the full-length human genomic α sequence in CLH3AXSV2DHFR with a partial genomic TSHβ sequence in CLH3AXSV2ODC. The partial genomic TSHβ fragment used in this experiment consisted of the protein coding regions of exon II and III, and the 0.5 kb intron which separated the two exons (Table 9). All 5' and 3' regions flanking the protein coding sequence of TSHβ were removed in this particular construction. Following cotransfection with the two expression vectors, stable cell lines were cultured and analyzed for their ability to express TSH. Expression levels of the diner, as determined by a sensitive and specific radioimmunoassay, are listed in Table 3. Previous studies with the transfection of the cDNA for the α subunit with the genomic TSHβ clone had demonstrated that expression levels were usually below the sensitivity of the radioimmunoassay, usually less than 0.02 pg/cell/24h.

**TABLE 3**

| TSH Cell Line | Expression Rate pg/cell/24h |
|---|---|
| 7 | 0.19 |
| 8 | 0.22 |
| 10 | 0.24 |
| 12 | 0.24 |
| 37 | 0.13 |
| 48 | 2.97 |

TSH production, then, like FSH production, can be greatly enhanced by use of the full genomic α sequence, rather than the cDNA sequence, in mammalian cell transfections. In this experiment the range of TSH production enhancement was 6 to 100 fold.

### Example 4: Introns and Expression Enhancement

The human α genomic construction differed from the human α cDNA construction not only in that it contained introns, but also in that it contained endongenous 5' untranslated sequence, the endogenous polyadenylation signal, and additional 3' flanking sequences. Therefore, one could not infer from the results of the previous set experiments which genomic regions contributed to the enhanced expression.
A. To determine if the introns within the genomic α sequence were responsible for the elevated α subunit levels, we inserted a 2 kb XbaI-PstI portion of the human α intron A between the mouse MT-I 5' untranslated region and the α cDNA sequence in the CLH3AXSV2TPA vector (Figure 2). The truncated intron retained the endogenous splice acceptor, but the splice donor was supplied by a synthetic oligonucleotide.
B. Another plasmid was constructed to test the effect of a heterologous intron on α subunit expression. In this construction, a 130 bp intron from the MOPC41 immunoglobulin K gene (5) was inserted between the mouse MT-I 5' untranslated region and the α cDNA sequence. No transcriptional enhancer elements were included in this particular intervening sequence.
C. The intron-containing α cDNA constructs were compared to the original α cDNA construct and the full length genomic α sequence by transient transfection of COS-7 cells with the plasmid DNA and analysis of mRNA levels by northern blotting. In this experiment, the tissue plasminogen activator (tPA) cDNA served as an internal standard and was used to correct for the variations in the transfection efficiency of different plasmid constructions and thus normalize measured α subunit mRNA levels. Transfections were done in duplicate using the DEAE-dextran protocol of Seed and Aruffo (28). Two days after transfection, total cellular RNA was isolated from the cells. The RNA (5 micrograms) was fractionated on formaldehyde gels and then transferred to nylon membranes using standard northern blotting techniques. The membranes were then hybridized to either a ³²P-labeled human α or a ³²P-labeled tPA probe and the resulting signals were quantitated on a Betascope Model 603 blot analyzer (Betagen Corp., Waltham, MA). Normalized α mRNA values for relative comparisons were calculated by dividing the number of α counts by the number of tPA counts and then averaging the numbers obtained for duplicate samples. Results of the experiment are shown in Table 4.

**TABLE 4**

| Plasmid Construction | | Betascope counts | | Normalized Values (α/tPA) | Average of Duplicates |
|---|---|---|---|---|---|
| | | α probe | tPA probe | | |
| 1. α genomic | A | 6315 | 958 | 6.6 | 7 |
| | B | 7836 | 1177 | 6.7 | |
| | | | | | |
| 2. α cDNA + α IVS | A | 25353 | 1294 | 19.6 | 21 |
| | B | 34616 | 1559 | 22.2 | |
| | | | | | |
| 3.α cDNA + Ig IVS | A | 31743 | 1690 | 18.8 | 17 |
| | B | 37495 | 2327 | 16.1 | |
| | | | | | |
| 4.α cDNA | A | 3891 | 2608 | 1.5 | 1 |
| | B | 3671 | 3341 | 1.1 | |

The results indicated that the cDNA constructions which contained either the human α intron (No.2) or the immunoglobulin intron (No. 3) were more efficient as the full length genomic sequence (No. 1) in the accumulation of α subunit mRNA. Specifically, the normalized α subunit mRNA levels were 7- to 21-fold higher than those produced by the cDNA construct (No. 4) without an intron. We have therefore unexpectedly discovered that introns, and not the 5' or 3' regions which flank the α subunit protein coding sequence, are primarily responsible for the increased expression levels and that the effect is due, at least in part, to an increased accumulation of α subunit mRNA.

### Example 5: Expression Enhancement Not Due to Increased Transcription Rates.

Nuclear runoff transcription assays were used to determine if the high level of genomic α-induced mRNA accumulation was due to an increased transcription rate. Transient transfection of COS-7 cells with the α genomic and α cDNA-containing CLH3AXSV2TPA plasmids was done as described in Example 4. Standard methods were used in the preparation of nuclei and for the runoff transcription assay (Current Protocols in Molecular Biology; Ausubel, F. M. et al., ed.; John Wiley & Sons, NY, NY). The DNA probes for α, DHFR, and tPA were gel purified insert sequences and approximately 0.25 µg of each were slot blotted in duplicate onto nitrocellulose membranes. The membranes were hybridized to [³²P-UTP] labeled nuclear runoff RNA prepared from COS-7 cells that had been transfected with no DNA (mock), the full-length human α genomic clone in CLH3AXSV2TPA, or the human α cDNA clone in CLH3AXSV2TPA. The hybridization signal was quantitated on a Betascope Model 603 blot analyzer (Betagen, Corp., Waltham, MA). Normalized values for the transcription rate were obtained by dividing the averaged α counts by the averaged tPA counts. The monkey DHFR nuclear runoff RNA should not hybridize to the mouse DHFR DNA probe sequence in the conditions used for this experiment, and therefore serves as a negative control. The relative transcription rate results are summarized in Table 5, below.

**TABLE 5**

| Nuclear runoff RNA | | | | | | |
|---|---|---|---|---|---|---|
| DNA Probes | Mock COS-7 | | α cDNASV2TPA | | αgenomic/SV2TPA | |
| | 1 | 2 | 1 | 2 | 1 | 2 |
| α cDNA | 119 | 160 | 2093 | 2155 | 492 | 620 |
| tPA | 387 | 413 | 18022 | 17667 | 8486 | 7388 |
| DHFR | 230 | 307 | 54 | 0 | 32 | 765* |
| | | | | | | |
| Rate Ratios: α/tPA | - | | 0.12 | | 0.07 | |

| | | | | | | |
|---|---|---|---|---|---|---|
| *Non-specific background rendered this value artifically high | | | | | | |

In this particular experiment the runoff transcription rate of the α cDNA (0.12) was surprisingly higher than that of the genonic α sequence (0.07). An increased transcription rate is therefore not the mechanism by which the genomic α sequence generates higher α subunit expression levels.

### Example 6: TSHβ Subunit Expression is Intron-dependent.

A. The full-length genomic TSHβ gene (14a) is diagrammed in Table 3, Part A. The positions of the three exons (I, II, and III), two introns (a, b), start ATG, and stop codon (TAA) are indicated. The PvuII sites were separated by approximately 2 kb of DNA sequence which included exons II and III and contained the complete protein coding sequence for TSHβ. The TSHβ partial genomic constructions used in this study were derived from the 2 kb PvuII fragment and are diagrammed in Table 3B. TSHβ 0.9 (0.9 kb) was the same construct used in stable transfections in Example 1 and consisted of the two coding exons separated by the endogenous intervening sequence with all sequences upstream of the start ATG and downstream of the TAA removed. TSHβ 1.2 (1.2 kb) and TSHβ 2.0 (2.0 kb) contained, in addition, about 300 base pairs of intron A and were constructed by adding a synthetic splice donor to allow splicing of the truncated intron. TSHβ 2.0 retained the endongenous polyadenylation signal and about 0.8 kb of additional 3' flanking sequence.
B. Duplicate cultures (A, B) of COS-7 cells were transfected, using the protocol described in Example 4, with CLH3AXSV2DHFR plasmids which contained one of the following partial genomic fragments: (1) TSHβ 0.9, (2) TSHβ 1.2, or (3) TSHβ 2.0. The genes were inserted into the XhoI cloning site so that transcription would be initiated by the MT-I promoter and, in the case of TSHβ 0.9 and TSHβ 1.2, terminated by the SV40 early polyadenylation signal. After 48 hours of incubation, total cellular RNA was isolated from the cells. The RNA (9 micrograms) was fractionated on formaldehyde gels and then transferred to nylon membranes using standard northern blotting techniques. The membranes were hybridized to either a ³²P-labeled mouse MT-I probe (to detect the TSHβ mRNA which also contains about 50 base pairs MT-I 5' untranslated sequence) or a ³²P-labeled DHFR probe (to compare transfection efficiencies). The resulting signals were quantitated on a Betascope Model 603 blot analyzer (Betagen Corp., Waltham, MA). Normalized TSHβ mRNA values for relative comparisons were calculated by dividing MT-I counts by DHFR counts. Normalized values obtained for duplicate transfections were then averaged and divided by the number obtained for TSHβ 0.9. The comparative results obtained for accumulation of mRNA are summarized in Table 6.

**TABLE 6**

| Plasmid Construction | | Betascope Counts | | Normalized Values (MT/DHFR) | Relative Values (averaged) |
|---|---|---|---|---|---|
| | | MT Probe | DHFR Probe | | |
| 1. TSEβ 0.9 | A | 983 | 3985 | 0.25 | 1 |
| | B | 834 | 4118 | 0.20 | |
| | | | | | |
| 2. TSHβ 1.2 | A | 4480 | 254 | 17.6 | 60 |
| | B | 2748 | 285 | 9.6 | |
| | | | | | |
| 3. TSEβ 2.0 | A | 1381 | 1209 | 1.2 | 5 |
| | B | 1632 | 1254 | 1.3 | |

C. The results indicated that the two constructs which retained a portion of the first intron (TSEβ 1.2 and TSHβ 2.0) yielded 5-60 fold higher mRNA accumulation than did the construct which contained no sequences from the first intron. Therefore TSHβ gene expression, like α subunit gene expression, is intron-dependent. Of interest was the unexpected observation that intron B, which was present in all the constructs and was in the protein coding region, did not confer optimal enhancement. This suggested either that there are specific sequences in the first intron of the TSHβ gene which increased mRNA accumulation or, more likely, that the position of the intron (close to the 5' end of the mRNA) was the crucial factor. That intron-dependent gene expression may be affected by the placement of the intron in the transcription unit is supported by preliminary experiments with the human α cDNA. Insertion of the K immunoglobulin gene intron in the 3' untranslated region of the mRNA, between the α cDNA and the SV40 polyadenylation region, resulted in no increased mRNA accumulation when compared to the unaltered α cDNA.
D. Since TSHβ 1.2 yielded 10-fold higher amounts of mRNA than TSEβ 2.0, it is probable that the endogenous polyadenylation signal and 3' flanking sequence carried on TSHβ 2.0 are not required for efficient mRNA formation, and may actually be detrimental. Alternatively, the disparity in mRNA accumulation between TSHβ 1.2 and TSHβ 2.0 may be related to the different distances between the MT-I promoter and the SV40 early promoter (driving the DHFR) in the two plasmid constructions. The closer proximity (0.8 kb) of the two promoters in the TSHβ 1.2 construction may permit increased interaction between the SV40 enhancer elements and the MT-I promoter, thereby increasing the rate of transcription from the latter.

Because of the similarity of α and TSHβ gene structure among various mammals, we expect that our findings based on the human α and TSHβ subunits apply equally to those of other species, such as the bovine, equine, porcine, baboon and monkey. Likewise, LHβ and FSHβ subunit genes may show a similar dependence on genomic DNA structure for optimal expression and these requirements should apply across species.

Construction of expression vectors will be advantageously simplified by application of the knowledge that certain genomic regions, such as introns, are important for mRNA accumulation, and that the position of these regions in the transcription unit may be important. For example, these genomic regions may be subcloned or synthesized and included in constructions with the α and β subunit cDNA sequences. The cDNA clones are often easier to obtain and to engineer than the much larger genomic clones. Thus, this discovery will simplify the development of cell lines which produce high levels of hormone and, ultimately, will permit large scale production of these proteins at a lower cost. These and other aspects and variations of the present invention will now be readily apparent to those skilled in the art and are not deemed to depart from either the spirit or the scope of the present invention.

### References

1. Hamer, D.H. and Leder, P. (1979) Cell 17, 737-747
2. Hamer, D.H. and Leder, P. (1979) Cell 18, 1299-1302
3. Banerji, J. et al. (1983) Cell 33, 729-740
4. Gillies, S.D. et al. (1983) Cell 33, 717-728
5. Queen, C. and Baltimore, D. (1983) Cell 33, 741-748
6. Gross, M. K. et al. (1987) Mol. Cell. Biol. 7, 4576-4581
7. Kaetzel, D.M. and Nilson, J.H. (1988) J. Biol. Chem. 263, 6344-6351
8. Fiddes, J.C. and Goodman, H.M. (1981) J. Mol. Appl. Genetics 1, 3-18
9. Goodwin, R.G. et al. (1983) Nucl. Acids Res. 11, 6873-6882
10. Virgin, J. et al. (1985) J. Biol. Chem. 260, 7072-7077
11. Talmadge, K. et al. (1984) Nature 307, 37-40
12. Kim, K.E. et al. (1988) DNA 7, 227-233
13. Watkins, P.C. et al. (1987) DNA 6, 205-212
14. Wondisford, F.E. et al. (1988) Mol. Endo. 2, 32-39
14a. Wondisford, F.E. et al. (1988) J. Biol. Chem. 263, 12538-12542
15. Ramabhadran, T.V. et al. (1984) Proc. Natl. Acad. Sci. USA 81, 6701-6705
16. Reddy, V.B. et al. (1985) Proc. Natl. Acad. Sci. USA 82, 3644-3648
17. Watanabe, S. et al. (1987) Biochem. Biophys. Res. Comm. 149, 1149-1155
18. Matzuk, M.M. et al. (1987) Proc. Natl. Acad. Sci. USA 84, 6354-6358
18a. Matzuk, M. and Boime, I. (1988) J. Cell Biol. 106, 1049-1059
19. Kaetzel, D.M. et al. (1985) Proc. Natl. Acad. Sci. USA 82, 7280-7283
20. Buchman, A.R. and Berg, P. (1988) Mol. Cell. Biol. 8, 4395-4405
21. Lee, F. et al. (1981) Nature 294, 228-232
22. Evans, M.J. and Scarpulla, R.C. (1988) Mol. Cell. Biol. 8, 35-41
23. Rossouw, C.M.S. et al. (1987) J. Biol. Chem. 262, 15151-15157
24. Brinster, R.L. et al. (1987) Proc. Natl. Acad. Sci. USA 85, 836-840
25. Pfarr, D.S. et al. (1986) DNA 5, 115-122
26. Ross, J. and Kobs, G. (1986) J. Mol. Biol. 188, 579-593
27. Kozak, M. (1984) Nucl. Acids Res. 12, 857-872
28. Seed, B. and Aruffo, A. (1987) Proc. Natl. Acad. Sci. USA 84, 3365-3365
29. Bolivar, F., Rodriguez, R.L., Green, P.J., Betlach, M.C., Heynecker, H.L., Boyer, H.V., Crosa, G.F. and Falkow, S. (1977) Gene 2:95.
30. Glanville, N., Durnham, D.M. and Palmiter, R.D. (1981) Nature 292:267-269.
31. Hamer, D.H. and Walling, M. (1982) J. Mol. Appl. Gen. 1:273-288.
32. Lusky, M. and Botchan, M. (1981) Nature 283:79-81.
33. Reddy, V.B., Thimmapaya, B., Dhar, R., Subramanian, K.N., Zain, B.S., Pan, J., Ghosh P.K., Celma, M.L. and Weissman, S.M. (1978) Science 200:494.
34. Tooze, J. (ed.), DNA Tumor Viruses; Molecular Biology of Tumor Viruses; 2nd Ed., Cold Spring Harbor Laboratory, New York 1981.
35. Chiang T.-R. and McConlogue, L. (1988), Mol. Cell. Biol. 8:764-769.

Except as otherwise stated, procedures and techniques used are generally as reported and conventional in the art such as is described by Maniatis et al, A Cloning Manual, Cold Spring Harbor (1982). All references cited above or in the specification are fully incorporated herein by reference.

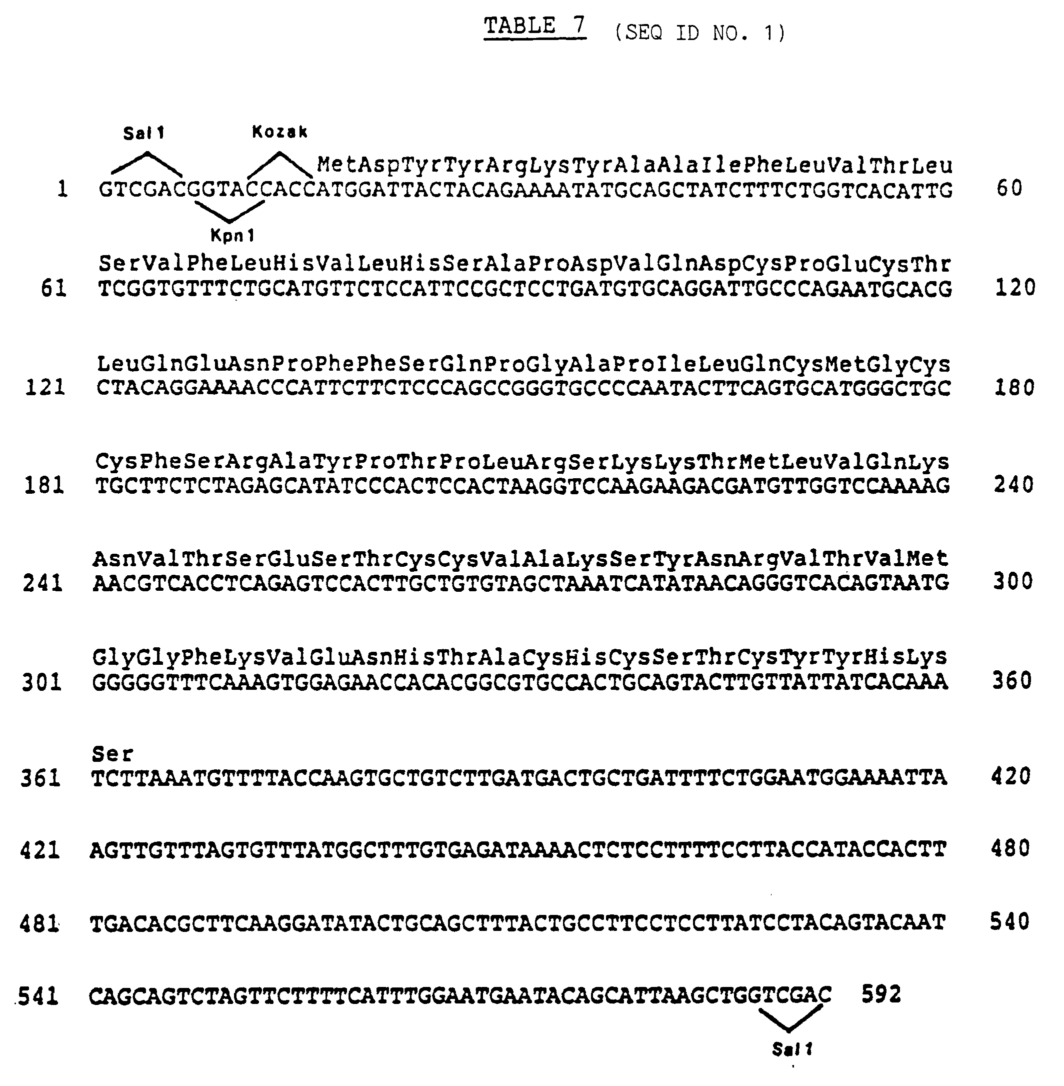

The engineered alpha subunit cDNA sequence. Restriction endonuclease sites and Kozak consensus region which were supplied by synthetic oligonucleotides are indicated. The alpha subunit amino acid sequence is also shown.

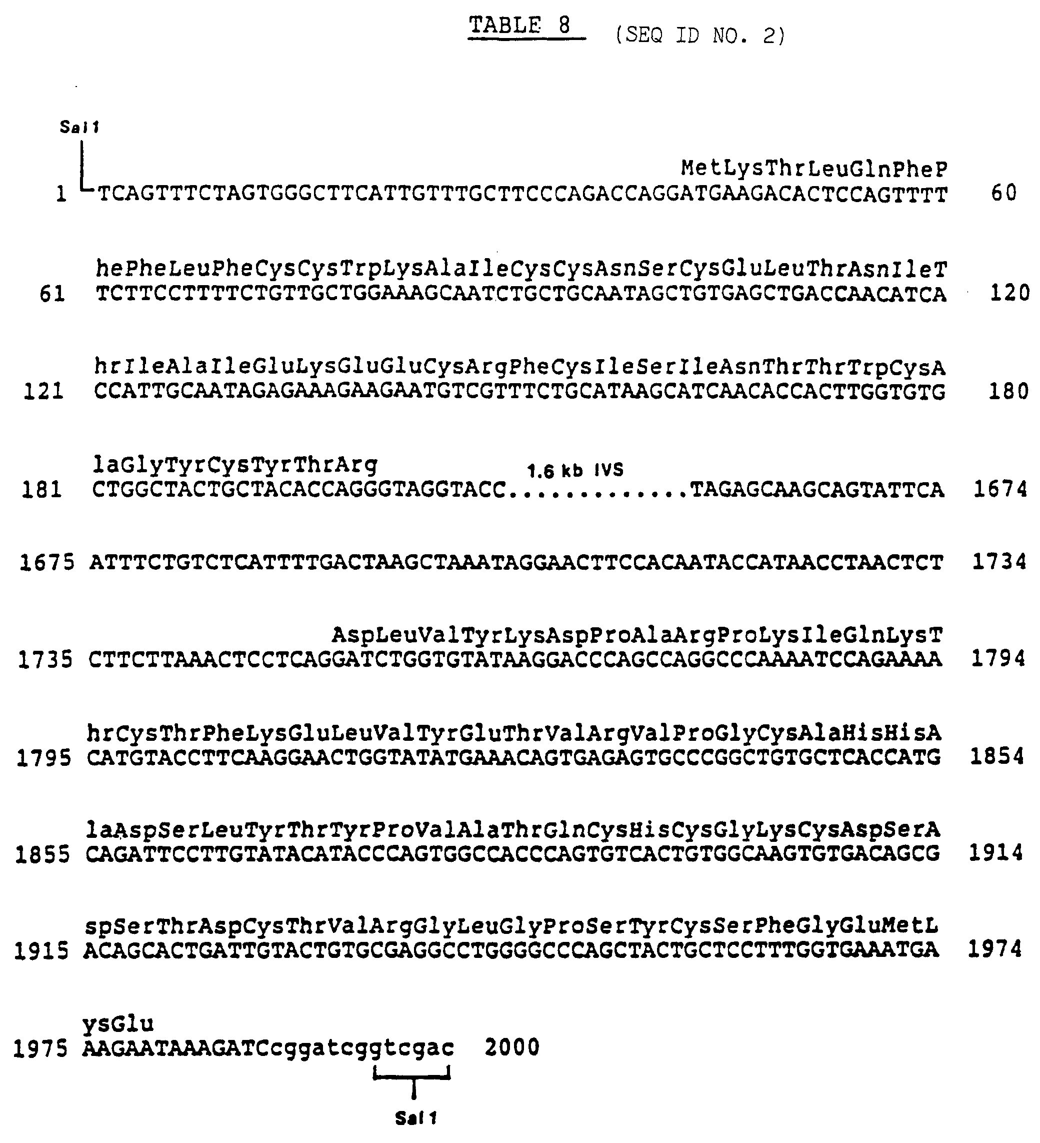

The engineered human FSH beta subunit partial genomic sequence. Positions of the terminal SalI sites are indicated. Nucleotides in lower case at the 3' terminus results from attachment of synthetic linkers. The FSH beta amino acid sequence marks the coding regions of exons II and III. The dotted line represents uncharacterized intervening sequence (IVS).

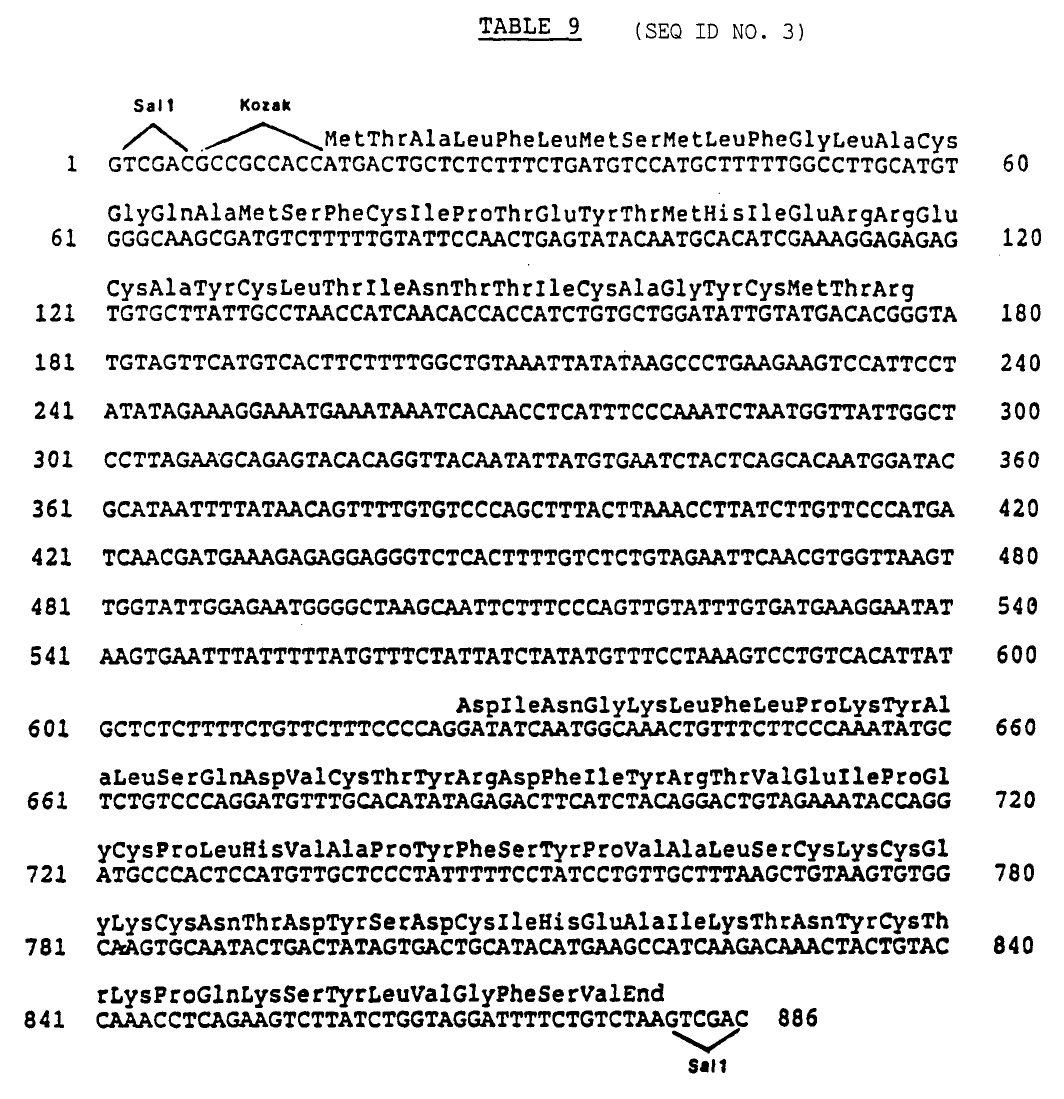

The engineered TSH beta subunit partial genomic sequence. SalI sites and Kozak consensus sequence supplied by synthetic oligonucleotides are indicated. The TSH beta amino acid sequence marks the coding regions of exons II and III.

### SEQUENCE LISTING:

**SEQ ID N0: 1**
   **SEQUENCE TYPE:** Nucleotide with corresponding protein
   **SEQUENCE LENGTH:** 592 base pairs
**FEATURES:**
   from 1 to 6 bp Sail site
   from 7 to 12 bp KpnI site
   from 11 to 15 bp Kozak consensus region
   from 587 to 592 bp SalI site
**PROPERTIES:** Engineered cDNA sequence of alpha subunit of dimeric glycoprotein hormones.
**SEQUENCE:** 
GTCGACGGTA CCACC ATG GAT TAC TAC AGA AAA TAT GCA GCT 42
Met Asp Tyr Tyr Arg Lys Tyr Ala Ala
1               5

ATC TTT CTG GTC ACA TTG TCG GTG TTT CTG CAT GTT CTC 81
Ile Phe Leu Val Thr Leu Ser Val Phe Leu His Val Leu
10                  15                  20

CAT TCC GCT CCT GAT GTG CAG GAT TGC CCA GAA TGC ACG 120
His Ser Ala Pro Asp Val Gln Asp Cys Pro Glu Cys Thr
        25                  30                  35

CTA CAG GAA AAC CCA TTC TTC TCC CAG CCG GGT GCC CCA 159
Leu Gln Glu Asn Pro Phe Phe Ser Gln Pro Gly Ala Pro
                40                  45

ATA CTT CAG TGC ATG GGC TGC TGC TTC TCT AGA GCA TAT 198
Ile Leu Gln Cys Met Gly Cys Cys Phe Ser Arg Ala Tyr
    50                  55                  60

CCC ACT CCA CTA AGG TCC AAG AAG ACG ATG TTG GTC CAA 237
Pro Thr Pro Leu Arg Ser Lys Lys Thr Met Leu Val Gln
            65                  70

AAG AAC GTC ACC TCA GAG TCC ACT TGC TGT GTA GCT AAA 276
Lys Asn Val Thr Ser Glu Ser Thr Cys Cys Val Ala Lys
75                  80                  85

TCA TAT AAC AGG GTC ACA GTA ATG GGG GGT TTC AAA GTG 315
Ser Tyr Asn Arg Val Thr Val Met Gly Gly Phe Lys Val
        90                  95                  100

GAG AAC CAC ACG GCG TGC CAC TGC AGT ACT TGT TAT TAT 354
Glu Asn His Thr Ala Cys His Cys Ser Thr Cys Tyr Tyr
                105                 110

CAC AAA TCT TAAATGTTTT ACCAAGTGCT GTCTTGATGA 393
His Lys Ser
    115
 
SEQ ID NO: 1 (Cont'd)

CTGCTGATTT TCTGGAATGG AAAATTAAGT TGTTTAGTGT TTATGGCTTT 453
GTGAGATAAA ACTCTCCTTT TCCTTACCAT ACCACTTTGA CACGCTTCAA 493
GGATATACTG CAGCTTTACT GCCTTCCTCC TTATCCTACA GTACAATCAG 553
CAGTCTAGTT CTTTTCATTT GGAATGAATA CAGCATTAAG CTGGTCGAC 592

END OF SEQ ID NO: 1
**SEQ ID N0: 2**
   **SEQUENCE TYPE:** Nucleotide with corresponding protein
   **SEQUENCE LENGTH:** 2000 base pairs including uncharacterised intervening sequence
**FEATURES:**
   before 1 bp Sail site
   from 42 bp to 209 bp exon II
   from 210 to 1655 bp uncharacterised intervening sequence (IVS)
   from 1666 to 1979 bp exon III
   from 1988 to 2000 bp nucleotides resulting from attachment of synthetic linkers
   from 1995 to 2000 bp SalI site
**PROPERTIES:** Engineered human genomic sequence of beta subunit of follicle stimulating hormone (FSH)
**SEQUENCE:** 
TCAGTTTCTA GTGGGCTTCA TTGTTTGCTT CCCAGACCAG G ATG 44
Met
1

AAG AGA CTC CAG TTT TTC TTC CTT TTC TGT TGC TGG AAA 83
Lys Thr Leu Gln Phe Phe Phe Leu Phe Cys Cys Trp Lys
            5                   10

GCA ATC TGC TGC AAT AGC TGT GAG CTG ACC AAC ATC ACC 122
Ala Ile Cys Cys Asn Ser Cys Glu Leu Thr Asn Ile Thr
15                  20                  25

ATT GCA ATA GAG AAA GAA GAA TGT CGT TTC TGC ATA AGC 161
Ile Ala Ile Glu Lys Glu Glu Cys Arg Phe Cys Ile Ser
        30                  35                  40

ATC AAC ACC ACT TGG TGT GCT GGC TAC TGC TAC ACC AGG 200
Ile Asn Thr Thr Trp Cys Ala Gly Tyr Cys Tyr Thr Arg
                45                  50

GTAGGTACC..........TAGAGCAAGC AGTATTCAAT TTCTGTCTCA 1686
TTTTGACTAA GCTAAATAGG AACTTCCACA ATACCATAAC 1726
CTAACTCTCT TCTTAAACTC CTCAG GAT CTG GTG TAT AAG GAC 1769
Asp Leu Val Tyr Lys Asp
    55

CCA GCC AGG CCC AAA ATC CAG AAA ACA TGT ACC TTC AAG 1808
Pro Ala Arg Pro Lys Ile Gln Lys Thr Cys Thr Phe Lys
60                  65                  70

GAA CTG GTA TAT GAA ACA GTG AGA GTG CCC GGC TGT GCT 1847
Glu Leu Val Tyr Glu Thr Val Arg Val Pro Gly Cys Ala
        75                  80                  85

CAC CAT GCA GAT TCC TTG TAT ACA TAC CCA GTG GCC ACC 1886
His His Ala Asp Ser Leu Tyr Thr Tyr Pro Val Ala Thr
                90                  95
 
SEQ ID NO: 2 (Cont'd)

CAG TGT CAC TGT GGC AAG TGT GAC AGC GAC AGC ACT GAT     1925
Gln Cys His Cys Gly Lys Cys Asp Ser Asp Ser Thr Asp
    100                 105                 110

TGT ACT GTG CGA GGC CTG GGG CCC AGC TAC TGC TCC TTT     1964
Cys Thr Val Arg Gly Leu Gly Pro Ser Tyr Cys Ser Phe
            115                 120

GGT GAA ATG AAA GAA TAAAGATCCG GATCGGTCGA C             2000
Gly Glu Met Lys Glu
125

END OF SEQ ID NO: 2
**SEQ ID N0: 3**
   **SEQUENCE TYPE:** Nucleotide with corresponding protein
   **SEQUENCE LENGTH:** 886 base pairs
**FEATURES:**
   from 1 to 6 bp Sail site
   from 7 to 15 bp Kozak consensus sequence
   from 16 to 177 bp exon II
   from 626 to 878 bp exon II
**PROPERTIES:** Engineered genomic sequence of thyroid stimulting hormone (TSH) beta subunit.
**SEQUENCE:**

GTCGACGCCG CCACC ATG ACT GCT CTC TTT CTG ATG TCC ATG 42
Met Thr Ala Leu Phe Leu Met Ser Met
1               5

CTT TTT GGC CTT GCA TGT GGG CAA GCG ATG TCT TTT TGT 81
Leu Phe Gly Leu Ala Cys Gly Gln Ala Met Ser Phe Cys
10                  15                  20

ATT CCA ACT GAG TAT ACA ATG CAC ATC GAA AGG AGA GAG 120
Ile Pro Thr Glu Tyr Thr Met His Ile Glu Arg Arg Glu
        25                  30                  35

TGT GCT TAT TGC CTA ACC ATC AAC ACC ACC ATC TGT GCT 159
Cys Ala Tyr Cys Leu Thr Ile Asn Thr Thr Ile Cys Ala
                40                  45

GGA TAT TGT ATG ACA CGG GTATGTAGTT CATGTCACTT 197
Gly Tyr Cys Met Thr Arg
    50

CTTTTGGCTG TAAATTATAT AAGCCCTGAA GAAGTCCATT CCTATATAGA 247
AAGGAAATGA AATAAATCAC AACCTCATTT CCCAAATCTA ATGGTTATTG 297
GCTCCTTAGA AGCAGAGTAC ACAGGTTACA ATATTATGTG AATCTACTCA 347
GCACAATGGA TACGCATAAT TTTATAACAG TTTTGTGTCC CAGCTTTACT 397
TAAACCTTAT CTTGTTCCCA TGATCAACGA TGAAAGAGAG GAGGGTCTCA 447
CTTTTGTCTC TGTAGAATTC AACGTGGTTA AGTTGGTATT GGAGAATGGG 497
GCTAAGCAAT TCTTTCCCAG TTGTATTTGT GATGAAGGAA TATAAGTGAA 547
TTTATTTTTA TGTTTCTATT ATCTATATGT TTCCTAAAGT CCTGTCACAT 597
TATGCTCTCT TTTCTGTTCT TTCCCCAG GAT ATC AAT GGC AAA 640
Asp Ile Asn Gly Lys
55

CTG TTT CTT CCC AAA TAT GCT CTG TCC CAG GAT GTT TGC 679
Leu Phe Leu Pro Lys Tyr Ala Leu Ser Gln Asp Val Cys
60                  65                  70

ACA TAT AGA GAC TTC ATC TAC AGG ACT GTA GAA ATA CCA 718
Thr Tyr Arg Asp Phe Ile Tyr Arg Thr Val Glu Ile Pro
        75                  80                  85

SEQ ID NO: 3 (Cont'd)

GGA TGC CCA CTC CAT GTT GCT CCC TAT TTT TCC TAT CCT 757
Gly Cys Pro Leu His Val Ala Pro Tyr Phe Ser Tyr Pro
90 95

GTT GCT TTA AGC TGT AAG TGT GGC AAG TGC AAT ACT GAC 796
Val Ala Leu Ser Cys Lys Cys Gly Lys Cys Asn Thr Asp
100 105 110

TAT AGT GAC TGC ATA CAT GAA GCC ATC AAG ACA AAC TAC 835
Tyr Ser Asp Cys Ile His Glu Ala Ile Lys Thr Asn Tyr
115 120

TGT ACC AAA CCT CAG AAG TCT TAT CTG GTA GGA TTT TCT 874
Cys Thr Lys Pro Gln Lys Ser Tyr Leu Val Gly Phe Ser
125 130 135

GTC TAAGTCGAC 886
Val

END OF SEQ ID NO: 3

## Claims

1. A method for the production of a heteropolymeric dimeric protein selected from luteinising hormone, follicle stimulating hormone or chorionic gonadotropin, the method comprising the steps of:
a) providing at least one vector comprising a promoter, a structural gene encoding for at least a portion of the β subunit of said protein and optionally also at least a portion of the α subunit, and a terminating sequence; wherein the structural gene comprises an intron in a sequence coding for at least a portion of the β subunit of said protein and also an intron located 5' to the coding region for said at least a portion of the α subunit of said protein (if DNA encoding said at least a portion of the α subunit of said protein is present in the vector);
b) if the vector does not encode at least a portion of the α subunit, providing a second vector encoding said α subunit or a portion thereof and having an intron which is located 5' to the coding region for said α subunit or portion thereof;
c) transforming host cells with the vector(s) to thereby form stably transfected cells; and
d) culturing the transformed cells under conditions whereby said protein is produced;
with the proviso that said protein is not bovine luteinising hormone.

2. A method as claimed in claim 1 wherein one vector comprises a cDNA sequence encoding the α subunit.

3. A method as claimed in claim 2 wherein the α subunit comprises the sequence set forth in Table 7.

4. A method as claimed in claim 2 or 3 wherein the β subunit is part of a second vector.

5. A method as claimed in any of claims 2 to 4 wherein said cDNA encoding said α subunit comprises a plurality of introns, 3' and 5' flanking regions, endogenous 5' untranslated sequence and polyadenylation signal.

6. A method as claimed in claim 4 wherein the vector comprises genomic DNA encoding the α subunit and genomic DNA encoding the β subunit.

7. A method as claimed in claim 6, wherein part of the β subunit of FSH has the sequence shown in Table 8.

## Patentansprüche

1. Verfahren zur Herstellung eines heteropolymeren dimeren Proteins ausgewählt aus luteinisierendem Hormon, follikelstimulierendem Hormon oder Choriongonadotropin, wobei das Verfahren die Stufen umfasst:
a) dass mindestens ein Vektor mit einem Promotor, einem Strukturgen, das mindestens einen Teil der β-Untereinheit des Proteins und gegebenenfalls auch mindestens einen Teil der α-Untereinheit und eine terminierende Sequenz kodiert, bereitgestellt wird; wobei das Strukturgen ein Intron aufweist in einer Sequenz, die mindestens einen Teil der β-Untereinheit des Proteins kodiert und auch ein Intron aufweist, das 5' zur kodierenden Region für den mindestens einen Teil der α-Üntereinheit des Proteins angeordnet ist (wenn DNA, die mindestens einen Teil der α-Untereinheit des Proteins kodiert, in dem Vektor vorhanden ist);
b) wenn der Vektor nicht mindestens einen Teil der α-Untereinheit kodiert, ein zweiter Vektor bereitgestellt wird, der die α-Untereinheit oder einen Teil davon kodiert und ein Intron aufweist, das 5' zur kodierenden Region für die α-üntereinheit oder einen Teil davon angeordnet ist;
c) Wirtszellen mit dem Vektor/den Vektoren transformiert wird/werden, um dadurch stabil transfizierte Zellen zu bilden und
d) die transformierten Zellen unter solchen Bedingungen gezüchtet werden, unter denen das Protein erzeugt wird;
mit dem Vorbehalt, dass das Protein nicht luteinisierendes Hormon vom Rind ist.

2. Verfahren nach Anspruch 1, wobei ein Vektor eine cDNA-Sequenz aufweist, die die α-Untereinheit kodiert.

3. Verfahren nach Anspruch 2, wobei die α-Untereinheit die in Tabelle 7 angegebene Sequenz aufweist.

4. verfahren nach Anspruch 2 oder Anspruch 3, wobei die β-Untereinheit Teil eines zweiten Vektors ist.

5. Verfahren nach einem der Ansprüche 2 bis 4, wobei die cDNA, die die α-Untereinheit kodiert, eine Vielzahl von Introns, 3'- und 5'-flankierende Regionen, eine endogene 5'- untranslatierte Sequenz und ein Polyadenylierungssignal aufweist,

6. Verfahren nach Anspruch 4, wobei der Vektor genomische DNA aufweist, die die α-Untereinheit kodiert, und genomische DNA, die die β-Untereinheit kodiert.

7. Verfahren nach Anspruch 6, wobei ein Teil der β-Untereinheit von FSH die in Tabelle 8 gezeigte Sequenz hat.

## Revendications

1. Procédé de production d'une protéine dimérique hétéropolymère choisie parmi l'hormone lutéinisante, l'hormone de stimulation des follicules ou la gonadotropine chorionique, ledit procédé comprenant les étapes suivantes :
a) le fait de fournir aù moins un vecteur comprenant un promoteur, un gène structural codant au moins une portion de la sous-unité β de ladite protéine et de façon optionnelle également au moins une portion de la sous-unité α, et une séquence de terminaison ; dans lequel le gène structural comprend un intron dans une séquence codant pour au moins une portion de la sous-unité β de ladite protéine et également un intron situé en position 5' de la région codante de la portion au moins de la sous-unité α de ladite protéine (si l'ADN qui code au moins ladite portion de la sous-unité α de ladite protéine est présent dans le vecteur) ;
b) si le vecteur ne code pas au moins une portion de la sous-unité α, le fait de fournir un deuxième vecteur qui code ladite sous-unité α ou une portion de celle-ci et qui a un intron situé en 5' de la région codante de ladite sous-unité α ou une portion de ladite sous-unité ;
c) le fait de transformer des cellules hôtes avec le ou les vecteurs pour produire des cellules transfectées stables, et
d) le fait de mettre en culture les cellules transformées dans des conditions dans lesquelles ladite protéine est produite ; avec la restriction que ladite protéine n'est pas l'hormone lutéinisante bovine.

2. Procédé selon la revendication 1, dans lequel un vecteur comprend une séquence d'ADNc codant la sous-unité α.

3. Procédé selon la revendication 2 dans lequel la sous-unité α comprend la séquence décrite dans le Tableau 7.

4. Procédé selon l'une quelconque des revendications 2 ou 3, dans lequel la sous-unité β est une partie du second vecteur.

5. Procédé selon l'une quelconque des revendications 2 à 4, dans lequel ledit ADNc codant ladite sous-unité α comprend plusieurs introns, des régions flanquantes en 3' et 5', une séquence non traduite endogène en 5' et un signal de polyadénylation.

6. Procédé selon la revendication 4, dans lequel le vecteur comprend un ADN génomique codant la sous-unité α et un ADN génomique codant la sous-unité β.

7. Procédé selon la revendication 6, dans lequel une partie de la sous-unité β de l'hormone de stimulation des follicules présente la séquence décrite dans le Tableau 8.
